(19) Europäisches Patentamt
European Patent Office
Office européen des brevets

(11) Veröffentlichungsnummer: **0 142 741**
**B1**

(12) # EUROPÄISCHE PATENTSCHRIFT

(45) Veröffentlichungstag der Patentschrift: **24.01.90**

(21) Anmeldenummer: **84112931.5**

(22) Anmeldetag: **26.10.84**

(51) Int. Cl.$^5$: **C 07 D309/06**, C 07 D321/06, C 07 D319/06, C 07 D319/12, C 07 D335/02, C 07 D339/06, C 07 D339/08, A 01 N 43/08, A 01 N 43/16, A 01 N 43/36, A 01 N 43/40

(54) **Cyclohexan-1,3-dionderivate, Verfahren zu ihrer Herstellung und ihre Verwendung zur Bekämpfung unerwünschten Pflanzenwuchses.**

(30) Priorität: **08.11.83 DE 3340265**

(43) Veröffentlichungstag der Anmeldung: **29.05.85 Patentblatt 85/22**

(45) Bekanntmachung des Hinweises auf die Patenterteilung: **24.01.90 Patentblatt 90/04**

(84) Benannte Vertragsstaaten: **AT BE CH DE FR GB IT LI LU NL SE**

(56) Entgegenhaltungen:
**EP-A- 0 070 370**
**EP-A- 0 071 707**
**Die Akte enthält technische Angaben, die nach dem Eingang der Anmeldung eingereicht wurden und die nicht in dieser Patentschrift enthalten sind.**

(73) Patentinhaber: **BASF Aktiengesellschaft**
**Carl-Bosch-Strasse 38**
**D-6700 Ludwigshafen (DE)**

(72) Erfinder: **Jahn, Dieter, Dr.**
**Burgunder Weg 8**
**D-6803 Edingen-Neckarhausen (DE)**
Erfinder: **Becker, Rainer, Dr.**
**Im Haseneck 22**
**D-6702 Bad Duerkheim (DE)**
Erfinder: **Keil, Michael, Dr.**
**Fontanestrasse 4**
**D-6713 Freinsheim (DE)**
Erfinder: **Richarz, Winfried, Dr.**
**Koenigsberger Strasse 5**
**D-6081 Stockstadt (DE)**
Erfinder: **Siegel, Hardo, Dr.**
**Hans-Purrmann-Allee 25**
**D-6720 Speyer (DE)**
Erfinder: **Spiegler, Wolfgang, Dr.**
**Westpreussenstrasse 5**
**D-6520 Worms 27 (DE)**
Erfinder: **Wuerzer, Bruno, Dr. Dipl.-Landwirt**
**Ruedigerstrasse 13**
**D-6701 Otterstadt (DE)**

**EP 0 142 741 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Die Erfindung betrifft herbizide Mittel, die Cyclohexan-1,3-dionderivate 5 als Wirkstoffe enthalten.

Es ist bekannt, Cyclohexan-1,3-dionderivate zur Bekämpfung unerwünschter Gräser in breitblättrigen Kulturen anzuwenden. Wirkstoffe mit Furyl- oder Thienylsubstituenten zeigen dabei eine relativ schwache Wirkung (DE-OS 24 39 104). Darüber hinaus sind aus EP-A-0 070 370, EP-A-0 071 707 und EP-A-0 136 702 heterocyclisch-substituierte Cyclohexan-1,3-dionderivate mit guter herbizider Wirkung gegen Pflanzen aus der Familie der Gräser bekannt.

Es wurde gefunden, daß herbizide Mittel, die Cyclohexan-1,3-dionderivate, die in 5-Stellung bestimmte heterocyclische Substituenten tragen, oder Salze dieser Verbindungen als Wirkstoffe enthalten, eine deutlich stärkere herbizide Wirkung gegen eine Reihe von Grasarten (Wild- und kulturarten) haben als die zum Stand der Technik gehörenden Herbizide. Dabei zeigen die erfindungsgemäßen Mittel eine exzellente Verträglichkeit sowohl für breitblättrige Kulturpflanzen als auch für monokotyle Kulturen, welche nicht zur Familie der Gräser zählen. Darüber hinaus ist ein Teil dieser Verbindungen trotz guter Wirkung gegen Gräser gleichzeitig für die Getreidekultur Weizen verträglich.

Die in den herbiziden Mitteln enthaltenen Cyclohexan-1,3-dionderivate haben die Formel

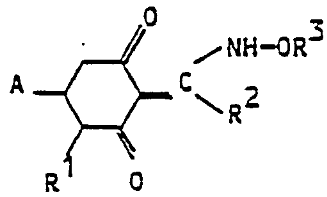

(I)

in der

A Tetrahydropyran-4-yl, 3-Methyltetrahydropyran-4-yl, 1,4-Dioxanyl, 5,5-Dimethyl-1,3-dioxan-2-yl, 2,5-Dimethyl-1,4-dioxan-3-yl, 2,6-Dimethyl-1,4-dioxan-3-yl, 2-Methyl-1,3-dithiolan-2-yl, 2,6-Dimethyltetrahydrothiopyran-3-yl, 2-Methyl-1,3-dithian-2-yl oder gegebenenfalls durch Alkyl mit 1 bis 8 Kohlenstoffatomen, Alkylen mit 4 oder 5 Kohlenstoffatomen oder gegebenenfalls methylsubstituiertes Cycloalkyl oder Bicycloalkyl mit 6 bis 12 Kohlenstoffatomen substituiertes 1,3-Dioxepan-5-yl,

$R^1$ Wasserstoff oder Methoxycarbonyl,

$R^2$ Alkyl mit 1 bis 4 Kohlenstoffatomen und

$R^3$ Alkyl mit 1 bis 4 C-Atomen, Alkenyl mit 3 oder 4 C-Atomen, Halogenalkenyl mit 3 oder 4 C-Atomen und 1 bis 3 Halogensubstituenten oder Propargyl bedeuten.

Die Verbindungen der Formel I können in mehreren Formen auftreten, die alle vom Patentanspruch umfaßt werden :

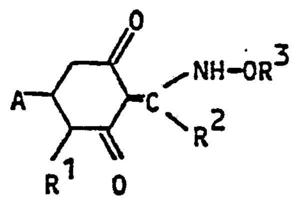

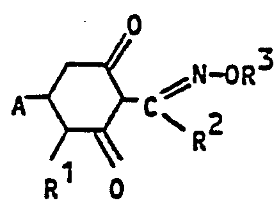

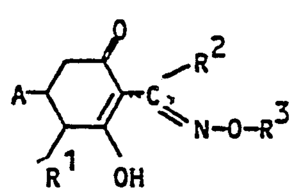

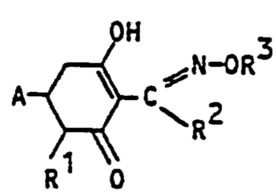

Die 1,3-Dioxepan-5-ylreste für A in Formel I können durch unverzweigte oder verzweigte Alkylreste mit 1 bis 8 Kohlenstoffatomen, wie Methyl, Ethyl, i-Propyl, t-Butyl, 1,2-Dimethyl-4-butyl, 1-Ethyl-n-pentyl oder durch Cycloalkyl oder Bicycloalkyl mit 6 mit 12 Kohlenstoffatomen, wie Cyclohexyl, Cycloheptyl, Cyclooctyl, Cyclododecyl, Bicycloheptyl, z.B. Bicyclo[3.1.1]-heptyl, substituiert sein. Die Cycloalkul- und Bicycloalkylreste können außerdem einen oder mehrere Metylsubstituenten tragen. Weiterhin können die 1,3-Dioxepan--5-ylreste durch eine Alkylenkette mit 4 oder 5 Kohlenstoffatomen, d.i. Tetramethylen oder Pentamethylen, substituiert sein, so daß beispielsweise Spiroverbindungen gebildet werden.

$R^2$ in Formel I steht für unverzweigte order verzweigte Alkylreste mit 1 bis 4 Kohlenstoffatomen, d. h. für Methyl, Ethyl, n-Propyl, i-Propyl, n-Butyl, sec.-Butyl, i-Butyl, tert. Butyl.

Reste für $R^3$ in Formel I sind Propargyl, Alkyl mit 1 bis 4 Kohlenstoffatomen, Alkenyl mit 3 oder 4 C-Atomen oder Halogenalkenyl mit 3 oder 4 C-Atomen, das bis zu drei Halogensubstituenten, z.B. Chlor,

Brom, Fluor, enthalten kann, beispielsweise Methyl, Ethyl, n-Propyl, i-Propyl, n-Butyl, sec.-Butyl, i-Butyl, tert.-Butyl, Allyl, 1-Chlorprop--1-en-3-yl, 2-Chlorprop-1-en-3-yl, 1,3-Dichlorprop-1-en-3-yl, 1,2,3-Trichlorpror-1-en-3-yl.

Bevorzugte Cyclohexan-1,3-dionderivate der Formel I sind solche, bei denen $R^1$ Wasserstoff bedeutet, und solche, bei denen $R^2$ Alkyl mit 2 oder 3 Kohlenstoffatomen bedeutet. Bevorzugte reste für A sind Tetrahydropyran-4-yl, 2,6-Dimethyl-tetrahydrothiopyran-3-yl oder gegebenenfalls durch Alkyl mit 1 bis 8 Kohlenstoffatomen, insbesondere 1 bis 4 Kohlenstoffatomen, Alkylen mit 4 order 5 Kohlenstoffatomen oder gegebenenfalls methylsubstituiertes Cycloalkyl oder Bicycloalkyl mit 7 bis 12 Kohlenstoffatomen substituierte 1,3-Dioxepan-5-ylreste, insbesondere 1,3-Dioxepan-5-yl oder durch unverzweigte oder verzweigte Alkylreste mit 1 bis 4 Kohlenstoffatomen substituierte 1,3-Dioxepan-5-ylreste.

Als Salze der Verbindungen der Formel I kommen beispielsweise die Alkalimetallsalze, insbesondere die Kalium- oder Natriumsalze, Erdalkalimetallsalze, insbesondere Calcium-, Magnesium- oder Bariumsalze sowie Mangan-, Kupfer-, Zink- oder Eisensalze und Ammonium- und Phosphoniumsalze, beispielsweise Alkylammonium-, Dialkylammonium-, Trialkyl- oder Tetraalkylammoniumsalze, Benzyltrialkylammoniumsalze, Triphenylphosphoniumsalze, Trialkylsulfoniumsalze oder Trialkylsulfoxoniumsalze in Betracht.

Die Verbindung der Formel I können durch Umsetzung von Verbindungen der Formel

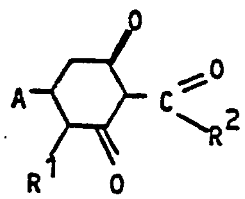

(II)

in der A, $R^1$ und $R^2$ die obengenannten Bedeutungen haben, mit Hydroxylaminderivaten $R^3O—NH_3Y$, in der $R^3$ die obengenannten Bedeutungen hat und Y ein Anion bedeutet, erhalten werden.

Man führt die Reaktion zweckmäßigerweise in heterogener Phase in einem inerten Verdünnungsmittel bei einer Temperatur zwischen 0 und 80 °C oder zwischen 0 °C und dem Siedepunkt des Reaktionsgemisches in Gegenwart einer Base durch. Geeignete Basen sind beispielsweise Carbonate, Hydrogencarbonate, Acetate, Alkoholate, Hydroxide oder Oxide von Alkali- oder Erdalkalimetallen, insbesonder von Natrium, Kalium, Magnesium, Calcium. Außerdem können auch organische Basen, wie Pyridin oder tertiäre Amine, Verwendung finden.

Als Lösungsmittel sind beispielsweise Dimethylsulfoxid, Alkohole, wie Methanol, Ethanol, Isopropanol, Benzol, gegebenenfalls chlorierte Kohlenwasserstoffe, wie Chloroform, Dichlorethan, Hexan, Cyclohexan, Ester, wie Essigsäureethylester, Ether, wie Dioxan, Tetrahydrofuran, geeignet.

Die Umsetzung ist nach wenigen Stunden beendet, das Reaktionsprodukt kann dann durch Einengen der Mischung, Zugabe von Wasser und Extraktion mit einem unpolaren Lösungsmittel, wie Methylenchlorid und Abdestillieren des Lösungsmittels unter vermindertem Druck isoliert werden.

Die Verbindung der Formel I können außerdem durch Umsetzen der Verbindungen der Formel II mit Hydroxylaminen der Formel $R^3O—NH_2$, in der $R^3$ die obengenannten Bedeutungen hat, in inerten Verdünnungsmitteln bei einer Temperatur zwischen 0 °C und dem Siedepunkt des Reaktionsgemisches, insbesondere zwischen 15 und 70 °C, erhalten werden. Gegebenenfalls kann das Hydroxylamin als wäßrige Lösung eingesetzt werden.

Geeignete Lösungsmittel für diese Umsetzung sind beispielsweise Alkohole, wie Methanol, Ethanol, Isopropanol, Cyclohexanol, gegebenenfalls chlorierte Kohlenwasserstoffe, wie Hexan, Cyclohexan, Methylenchlorid, Toluol, Dichlorethan, Ester, wie Essigsäureethylester, Nitrile, wie Acetonitril, cyclische Ether, wie Tetrahydrofuran.

Die Alkalimetallsalze der Verbindungen der Formel I können durch Behandeln dieser Verbindungen mit Natrium- oder Kaliumhydroxid in wäßriger Lösung oder in einem organischen Lösungsmittel, wie Methanol, Ethanol, Aceton, erhalten werden. Auch Natrium- und Kaliumalkoholate können als Basen dienen.

Die anderen Metallsalze, z.B. die Mangan-, Kupfer-, Zink-, Eisen-, Calcium-, Magnesium- und Bariumsalze, können aus den Natriumsalzen durch Reaktion mit den entsprechenden Metallchloriden in wäßriger Lösung hergestellt werden. Ammonium-, Sulfonium-, Sulfoxonium- und Phosphoniumsalze können durch Umsetzung von Verbindungen der Formel I mit Ammonium-, Phosphonium-, Sulfonium- und Sulfoxoniumhydroxiden, gegebenenfalls in wäßriger Lösung hergestellt werden.

Die Verbindungen der Formel II können aus Cyclohexan-1,3-dionen der Formel III, die auch in den tautomeren Formeln IIIa und IIIb vorliegen können,

(Siehe Formeln Seite 4 f.)

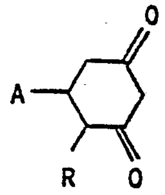

(III)

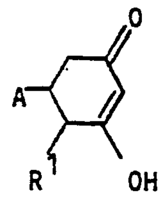

(IIIa)

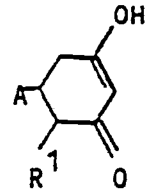

(IIIb)

nach literaturbekannten Methoden (Tetrahedron Letters, 29, 2491 (1975)) hergestellt werden.

Es ist auch möglich, Verbindungen der Formel II über die Zwischenstufe der Enolester, die bei der Umsetzung von Verbindungen der Formel III eventuell als Isomerengemische anfallen und in Gegenwart von Imidazol- oder Pyridinderivaten umgelagert werden (JP-OS 79/063052), herzustellen.

Zu den Verbindungen der Formel III gelangt man nach literaturbekannten Verfahren, wie dies aus folgendem Schema hervorgeht:

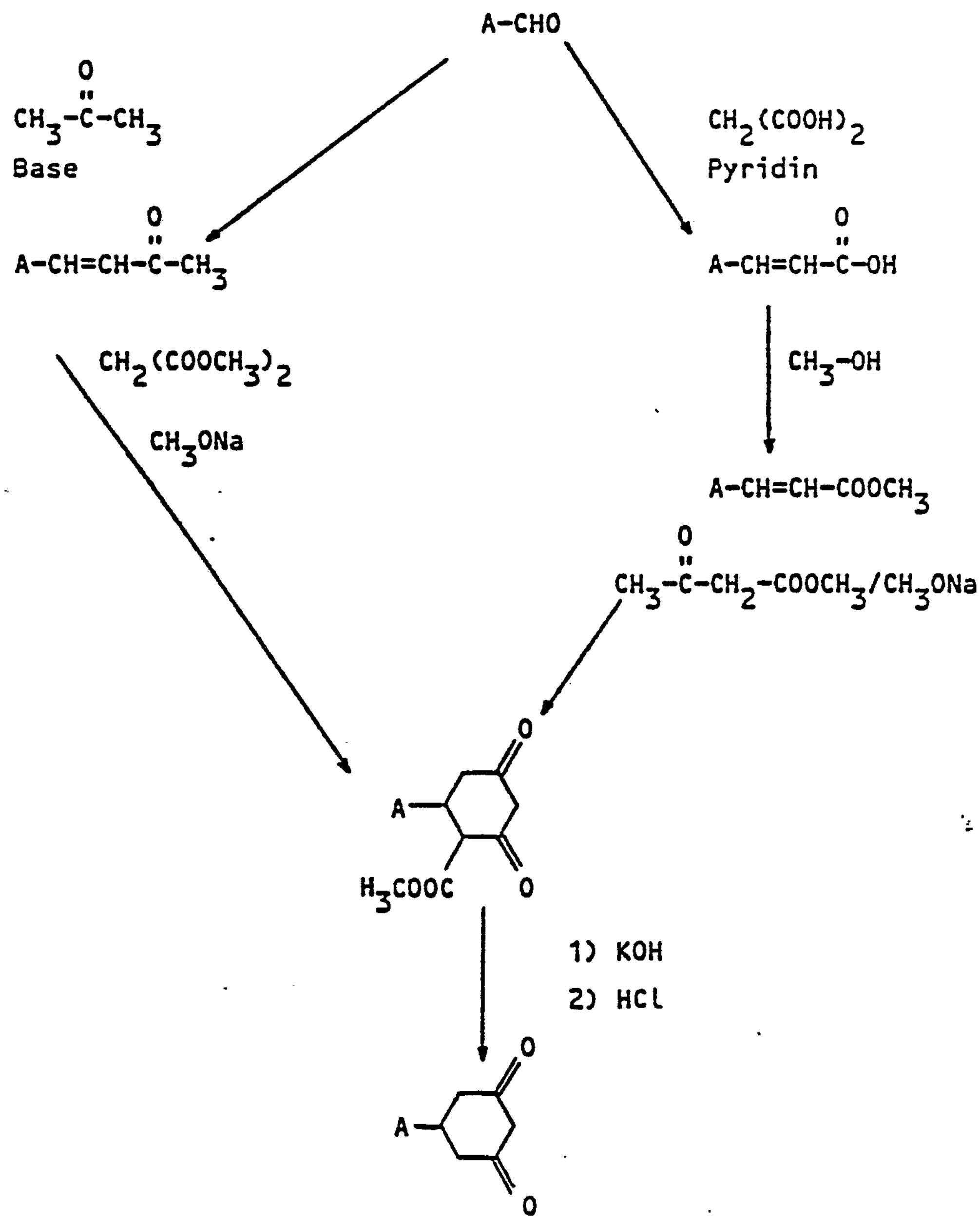


Zu Aldehyden der allgemeinen Formel A—CHO gelangt man nach literaturbekannten Verfahren, so

zum Beispiel durch Oxidation von entsprechenden Alkoholen, Reduktion von Carbonsäurederivaten, Hydroformylierung von Olefinen.

Die folgenden Beispiele erläutern die Herstellung der Cyclohexan-1,3-dionderivate der Formel I. In den Beispielen verhalten sich Gewichtsteile zu Volumenteilen wie Kilogramm zu Liter.

Die $^1$H-NMR-Spektren wurden in Deuterochloroform als Lösungsmittel mit Tetramethylsilan als innerem Standard aufgenommen. Die $^1$H-chemischen Verschiebungen sind jeweils in δ[ppm] angegeben. Für die Signalstruktur wurden folgende Abkürzungen benützt:

s = Singulett, d = Dublett, t = Triplett, q = Quartett, m = Multiplett, stärkstes Signal

## Beispiel 1

6,5 Gewichtsteile 2-Butyryl-5-(2-isopropyl-1,3-dioxepan-5-yl)-cyclohexan-1,3-dion, 2,1 Gewichtsteile Ethoxyammoniumchlorid und 1,8 Gewichtsteile Natriumhydrogencarbonat werden in 80 Volumenteilen Methanol 16 Stunden bei Raumtemperatur gerührt. Das Lösungsmittel wird unter vermindertem Druck abdestilliert, der Rückstand mit je 50 Volumenteilen Wasser und Dichlormethan gerührt, die organische Phase abgetrennt, die wäßrige Phase einmal mit 50 Volumenteilen Dichlormethan extrahiert, die vereinigten organischen Phasen werden über Natriumsulfat getrocknet, und das Lösungsmittel wird unter vermindertem Druck abdestilliert. Man erhält 6,2 Gewichtsteile 2-(1-Ethoxyamino-n-butyliden)-5-(2-isopropyl-1,3-dioxepan-5-yl)-cyclohexan-1,3-dion als öl. (Wirkstoff Nr. 1)

$n^{26}_D$ : 1,514

$^1$H-NMR-Spektrum :

δ = 0,90 (d), 1,18 (t), 2,45 (q), 4,15 (q)

## Beispiel 2

14,0 Gewichtsteile 2-Butyryl-5-(tetrahydropyran-4-yl)-cyclohexan-1,3-dion und 4,2 Gewichtsteile Allyloxyamin werden in 100 Volumenteilen Methanol bei Raumtemperatur 16 Stunden gerührt. Das Lösungsmittel wird unter vermindertem Druck abdestilliert, der Rückstand wird in Dichlormethan gelöst, die Lösung wird dann mit 5 % Niger Salzsäure und Wasser gewaschen, über Natriumsulfat getrocknet. Nach Abziehen des Lösungsmittels unter vermindertem Druck erhält man 12,9 Gewichtsteile 2-(1-Allyloxyamino-n-butyliden)-5-(tetrahydropyran-4-yl)-cyclohexan-1,3-dion als Feststoff. (Wirkstoff Nr. 2)

Fp. 55-56 °C

$^1$H-NMR-Spektrum

δ = 0,95 (t), 2,85 (t), 3,40 (t), 4,55 (d)

Die folgenden Verbindungen können in gleicher Weise erhalten werden :

(Siehe Tabellen Seite 6 ff.)

Die herbiziden Mittel, die die Cyclohexan-1,3-dionderivate der Formel I oder ihre Salze enthalten, können beispielsweise in Form von direkt versprühbaren Lösungen, Pulvern; Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie kerosin oder Dieselöl, ferner Kohlenteeröle usw., sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. aber auch Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphe-

| Wirkstoff-Nr. | A | $R^1$ | $R^2$ | $R^3$ | $^1$H-NMR-Daten (δ-Werte)/$n_D$/Fp. |
|---|---|---|---|---|---|
| 3 | 4-Tetrahydropyranyl | H | n-Propyl | Ethyl | Fp. 48 - 50°C |
| 4 | 4-Tetrahydropyranyl | H | Ethyl | Ethyl | 1,70 (d), 2,95 (q), 3,4 (t) |
| 5 | 4-Tetrahydropyranyl | H | Ethyl | Allyl | 1,15 (t), 2,90 (q), 4,55 (d) |
| 6 | 5,5-Dimethyl-1,3-dioxan-2-yl | H | n-Propyl | Allyl | 0,65 (s), 1,10 (s), 2,85 (m) |
| 7 | 5,5-Dimethyl-1,3-dioxan-2-yl | H | n-Propyl | Ethyl | Fp. 51°C |
| 8 | 2,6-Dimethyltetrahydrothio-pyran-3-yl | H | n-Propyl | Ethyl | |
| 9 | 2,6-Dimethyltetrahydrothio-pyran-3-yl | H | n-Propyl | Allyl | |
| 10 | 2,5-Dimethyl-1,4-dioxan--3-yl/2,6-Dimethyl-1,4-dioxan-3-yl (Isomerengemisch) | H | n-Propyl | Allyl | 0,98 (t), 1,07 (d), 2,90 (t) 4,55 (d) |
| 11 | 2,5-Dimethyl-1,4-dioxan--3-yl/1,6-Dimethyl-1,4-dioxan-3-yl (Isomerengemisch) | H | n-Propyl | Ethyl | 0,95 (t), 1,07 (d), 1,30 (t), 4,12 (g) |
| 12 | 1,4-Dioxanyl | H | n-Propyl | Ethyl | $n_D^{22}$ = 1,5226 |
| 13 | 1,4-Dioxanyl | H | n-Propyl | Allyl | $n_D^{22}$ = 1,5229 |
| 14 | 1,4-Dioxanyl | H | n-Propyl | 3-Chlor-allyl | $n_D^{22}$ = 1,5391 |
| 15 | 1,4-Dioxanyl | H | Ethyl | Ethyl | $n_D^{21}$ = 1,5259 |
| 16 | 1,4-Dioxanyl | H | Ethyl | Allyl | |
| 17 | 2-Isopropyl-1,3-dioxepan-5--yl | H | Ethyl | Ethyl | 0,90 (d), 1,14 (t), 1,32 (t) 2,45 (q), 4,30 (m) |
| 18 | 1,3-Dioxepan-5-yl | H | n-Propyl | Ethyl | 1,30 (t), 1,70 (m), 4,7 (s) |
| 19 | 1,3-Dioxepan-5-yl | H | n-Propyl | Allyl | 0,9 (t), 1,70 (m), 3,8 (m) |
| 20 | 1,3-Dioxepan-5-yl | H | Ethyl | Allyl | 1,15 (t), 1,70 (m), 2,9 (q) |
| 21 | 1,3-Dioxepan-5-yl | H | Ethyl | Ethyl | 1,7 (m), 3,8 (m), 4,65 (s) |

| Wirkstoff-Nr. | A | $R^1$ | $R^2$ | $R^3$ | $^1H$-NMR-Daten ($\delta$-Werte)/$n_D$/Fp. |
|---|---|---|---|---|---|
| 22 | 2-Methyl-1,3-dioxepan-5-yl | H | Ethyl | Ethyl | 1,1 (t), 1,7 (m), 2,9 (q) |
| 23 | 2-Methyl-1,3-dioxepan-5-yl | H | Ethyl | Allyl | |
| 24 | 2-Methyl-1,3-dioxepan-5-yl | H | n-Propyl | Ethyl | 0,9 (t), 4,1 (q), 4,9 (m) |
| 25 | 2-Methyl-1,3-dioxepan-5-yl | H | n-Propyl | Allyl | 0,9 (t), 2,65 (m), 4,5 (d) |
| 26 | 2-tert.-Butyl-1,3-dioxepan--5-yl | H | n-Propyl | Ethyl | 0,9 (s), 1,3 (t), 2,9 (m) |
| 27 | 2-tert.-Butyl-1,3-dioxepan--5-yl | H | n-Propyl | Allyl | |
| 28 | 2-tert.-Butyl-1,3-dioxepan--5-yl | H | Ethyl | Allyl | |
| 29 | 2-tert.-Butyl-1,3-dioxepan-5-yl | H | Ethyl | Ethyl | 0,9 (s), 1,15 (t), 1,7 (m) |
| 30 | 2-(1-Ethyl-n-pentyl)-di--1,3-oxepan-5-yl | H | Ethyl | Ethyl | |
| 31 | 2-(1-Ethyl-n-pentyl)-di--1,3-oxepan-5-yl | H | Ethyl | Allyl | |
| 32 | 2-(1-Ethyl-n-pentyl)-di--1,3-oxepan-5-yl) | H | n-Propyl | Allyl | |
| 33 | 2-(1-Ethyl-n-pentyl)-di-1,3-oxepan-5-yl) | H | n-Propyl | Ethyl | |
| 34 | 2-(1,2-Dimethyl-n-butyl)--1,3-dioxepan-5-yl | H | Ethyl | Ethyl | |
| 36 | 2-(1,2-Dimethyl-n-butyl)--1,3-dioxepan-5-yl | H | n-Propyl | Ethyl | |
| 37 | 2-(1,2-Dimethyl-n-butyl)--1,3-dioxepan-5-yl | H | n-Propyl | Ethyl | |

| Wirkstoff-Nr. | A | $R^1$ | $R^2$ | $R^3$ | $^1H$-NMR-Daten ($\delta$-Werte)/$n_D$/Fp. |
|---|---|---|---|---|---|
| 38 | 2-Cyclohexyl-1,3-dioxepan-5-yl | H | n-Propyl | Ethyl | |
| 39 | 2-Cyclohexyl-1,3-dioxepan-5-yl | H | n-Propyl | Allyl | |
| 40 | 2-Cyclohexyl-1,3-dioxepan-5-yl | H | Ethyl | Allyl | |
| 41 | 2-Cyclohexyl-1,3-dioxepan-5-yl | H | Ethyl | Ethyl | |
| 42 | 2-Cyclododecyl-1,3-dioxepan-5-yl | H | Ethyl | Ethyl | |
| 43 | 2-Cyclododecyl-1,3-dioxepan-5-yl | H | Ethyl | Allyl | |
| 44 | 2-Cyclododecyl-1,3-dioxepan-5-yl) | H | n-Propyl | Allyl | |
| 45 | 2-Cyclododecyl-1,3-dioxepan-5-yl) | H | n-Propyl | Ethyl | |
| 46 | 2-(2,6,6-Trimethylbicyclo-[3.1.1]-heptan-3-yl)-1,3--dioxepan-5-yl | H | n-Propyl | Ethyl | |
| 47 | 2-(2,6,6-Trimethylbicyclo-[3.1.1]-heptan-3-yl)-1,3-dioxepan-5-yl | H | n-Propyl | Allyl | |
| 48 | 2,2-Pentamethylen-1,3-dioxepan-5-yl | H | n-Propyl | Ethyl | 1,35 (t), 2,3 (m), 3,7 (m) |

| Wirkstoff-Nr. | A | $R^1$ | $R^2$ | $R^3$ | $^1H$-NMR-Daten ($\delta$-Werte)/$n_D$/Fp. |
|---|---|---|---|---|---|
| 49 | 2,2-Pentamethylen-1,3-dioxepan-5-yl | H | n-Propyl | Allyl | 1,6 (m), 4,55 (d), 6,0 (m) |
| 50 | 2-i-Propyl-1,3-dioxepan-5-yl | $COOCH_3$ | Ethyl | Allyl | |
| 51 | 2-i-Propyl-1,3-dioxepan-5-yl | $COOCH_3$ | Ethyl | Ethyl | |
| 52 | 2-Methyl-1,3-dithiolan-2-yl | H | n-Propyl | Ethyl | $n_D^{21}$ = 1,5705 |
| 53 | 2-Methyl-1,3-dithiolan-2-yl | H | n-Propyl | Allyl | $n_D^{21}$ = 1,5754 |
| 54 | 2-Methyl-1,3-dithiolan-2-yl | H | n-Propyl | 3-Chlorallyl | $n_D^{23}$ = 1,5829 |
| 55 | 2-Methyl-1,3-dithian-2-yl | H | n-Propyl | Ethyl | 0,98 (t), 1,32 (t), 1,60 (s), 4,11 (g) |
| 56 | 2-Methyl-1,3-dithian-2-yl | H | n-Propyl | Allyl | 0,96 (t), 1,60 (s), 4,50 (m), 5,30 (m), 5,90 (m) |
| 57 | 2-Methyl-1,3-dithian-2-yl | H | Ethyl | Allyl | 1,18 (t), 1,61 (s), 4,53 (d), 5,3 (m), 5,9 (m) |
| 58 | 2-Methyl-1,3-dithian-2-yl | H | Ethyl | 3-Chlorallyl | 1,13 (t), 1,60 (s), 4,63 (m), 6,2 (m) |
| 59 | 2-Methyl-1,3-dithian-2-yl | H | n-Propyl | 3-Chlorallyl | 0,96 (t), 1,58 (s), 4,60 (m) 6,20 (m) |
| 60 | 2-Methyl-1,3-dithian-2-yl | H | Ethyl | Ethyl | 1,18 (t), 1,35 (t), 1,62 (s), 4,11 (g) |

| Wirkstoff-Nr. | A | $R^1$ | $R^2$ | $R^3$ | $^1$H-NMR-Daten ( -Werte)/$n_D$/Fp. |
|---|---|---|---|---|---|
| 61 | 2-Isopropyl-1,3-dioxepan-5-yl | H | Ethyl | Allyl | 1,1 (t), 2,9 (q), 4,55 (d) |
| 62 | 2-Isopropyl-1,3-dioxepan-5-yl | H | Ethyl | Propargyl | 1,1 (t), 2,5 (s), 4,7 (d) |
| 63 | 2-Isopropyl-1,3-dioxepan-5-yl | H | Ethyl | 3-Chlor-allyl (trans) | 1,1 (t), 4,2 (m), 4,5 (d) |
| 64 | 2-Isopropyl-1,3-dioxepan-5-yl | H | Ethyl | 3-Chlor-allyl (cis) | 1,1 (t), 1,65 (d), 4,75 (d) |
| 65 | 2,2-Pentamethylen-1,3-dioxepan-5-yl | H | n-Propyl | Propargyl | 1,0 (t), 3,7 (m), 4,6 (m) |
| 66 | Tetrahydropyran-4-yl | H | n-Propyl | 3-Chlor-allyl (trans) | 0,95 (t), 2,8 (t), 4,5 (d) |
| 67 | Tetrahydropyran-4-yl | H | Ethyl | 3-Chlor-allyl (trans) | 1,1 (t), 3,4 (t), 4,5 (d) |
| 68 | 3-Methyl-tetrahydropyran-4-yl | H | Ethyl | Ethyl | |
| 69 | 3-Methyl-tetrahydropyran-4-yl | H | Ethyl | Allyl | |
| 70 | 3-Methyl-tetrahydropyran-4-yl | H | Ethyl | 3-Chlor-allyl (trans) | |

| Wirkstoff-Nr. | A | $R^1$ | $R^2$ | $R^3$ | $^1H$-NMR-Daten ($\delta$-Werte)/$n_D$/Fp. |
|---|---|---|---|---|---|
| 71 | 3-Methyl-tetrahydropyran-4-yl | H | n-Propyl | Ethyl | |
| 72 | 3-Methyl-tetrahydropyran-4-yl | H | n-Propyl | Allyl | |
| 73 | 3-Methyl-tetrahydropyran-4-yl | H | n-Propyl | 3-Chlor-allyl (trans) | |
| 74 | 5,5-Dimethyl-1,3-dioxan-2-yl | H | n-Propyl | Propargyl | 0,7 (s), 1,15 (s) |
| 75 | 5,5-Dimethyl-1,3-dioxan-2-yl | H | n-Propyl | 3-Chlor-allyl (cis) | 0,7 (s), 0,95 (t), 4,75 (d) |
| 76 | 5,5-Dimethyl-1,3-dioxan-2-yl | H | n-Propyl | 3-Chlor-allyl (trans) | 0,7 (s), 0,95 (t), 4,50 (d) |
| 77 | 5,5-Dimethyl-1,3-dioxan-2-yl | H | Ethyl | Ethyl | 1,3 (t), 4,10 (q), 4,35 (s) |
| 78 | 5,5-Dimethyl-1,3-dioxan-2-yl | H | Ethyl | Allyl | 1,2 (s), 4,35 (d), 4,5 (d) |
| 79 | 5,5-Dimethyl-1,3-dioxan-2-yl | H | Ethyl | 3-Chlor-allyl (cis) | 2,9 (m), 3,6 (d), 4,8 (d) |
| 80 | 5,5-Dimethyl-1,3-dioxan-2-yl | H | Ethyl | 3-Chlor-allyl (trans) | 1,1 (t), 3,4 (d), 4,55 (d) |
| 81 | 5,5-Dimethyl-1,3-dioxan-2-yl | H | Ethyl | Propargyl | 1,2 (s), 2,5 (s), 4,65 (d) |

nol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylaklkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesium-oxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die herbiziden Mittel enthalten zwischen 0,1 und 95 Gewichtsprozent, vorzugsweise zwischen 0,5 und 90 Gewichtsprozent, Wirkstoff.

Beispiele für herbizide Mittel sind :

I. Man vermischt 90 Gewichtsteile der Verbindung Nr. 2 mit 10 Gewichtsteilen N-Methyl-alphapyrrolidon und erhält eine Lösung, die zur Anwendung in Form kleinster Tropfen geeignet ist.

II. 20 Gewichtsteile der Verbindung Nr. 3 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-mono-ethanolamid, 5 Gewichtsteilen Calciumsalz der Dodecylbenzolsulfonsäure und 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 1 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteile des Anlagerungsproduktes von 40 Mol Ethylenoxid an 1 Mol Ricinusöl bestreht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile des Wirkstoffs Nr. 17 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewischtsteilen Wasser erhält man eine wäßrige Dispersion, die 0,02 Gewichtsprozent des Wirkstoffs enthält.

V. 20 Gewichtsteile ders Wirkstoffs Nr. 12 werden mit Gewichtsteilen des Natriumsalzes der Diisobutylnaphthalin-alpha-sulfonsäure, 17 Gewichtsteilen des Natriumsalzes einer Ligninsulfonsäure aus einer Sulfit-Abauge und 60 Gewichtsteilen pulverförmigen Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile des Wirkstoffs Nr. 52 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile des Wirkstoffs Nr. 67 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile des Wirkstoffs Nr. 6 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation kann im Vorauflauf- oder im Nachauflaufverfahren erfolgen. Sind die Wirkstoffe bei Nachauflaufanwendung für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,025 bis 3 kg/ha, vorzugsweise 0,05 bis 0,5 kg/ha.

Die Wirkung der erfindungsgemäßen herbiziden Mittel läßt sich durch Gewächshausversuche zeigen :

Als Kulturgefäße dienen Plastikblumentöpfe mit 300 $cm^3$ Inhalt und lehmigem Sand mit etwa 1,5 %

Humus als Substrat. Die Samen der Testpflanzen werden nach Arten getrennt flach eingesät.

Bei Vorauflaufbehandlung werden die Wirkstoffe unmittelbar danach auf die Erdoberfläche aufgebracht. Sie werden hierzu in Wasser als Verteilungsmittel suspendiert oder emulgiert und mittels fein verteilender Düsen gespritzt. Bei dieser Applikationsmethode betragen die Aufwandmengen 3,0 kg Wirkstoff/ha.

Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöle von 3 bis 15 cm an und behandelt sie danach. Die Sojapflanzen werden in einem mit Torfmull (peat) angereicherten Substrat angezogen. Zur Nachauflaufbehandlung werden entweder direkt gesäte und in den gleichen Gefäßen aufgewachsene Pflanzen ausgewählt, oder aber solche, die erst als Keimpflanzen getrennt angezogen und einige Tage vor der Behandlung in die Versuchsgefäße verpflanzt werden. Die Aufwandmengen für die Nachauflaufbehandlung variieren zwischen 0,03 und 0,25 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35 °C) und für solche gemäßigter Klimate 10 bis 25 °C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen :

Alopecurus myosuroides (Ackerfuchsschwanz), Avena fatua (Flughafer), Avena sativa (Hafer), Beta vulgaris (Zuckerrübe), Bromus inermis (Unbegrannte Trespe), Bromus spp. (Trespe), Digitaria sanguinalis (Blutfingerhirse), Echinochloa crus-galli (Hühnerhirse), Glycine max (Sojabohnen), Hordeum vulgare (Gerste), Lolium multiflorum (Ital. Raygras), Oryza sativa (Reis), Setaria italica (Kolbenhirse), Sinapsis alba (Weißer Senf), Triticum aestivum (Weizen), Zea mays (Mais), Sorghum halepense (Sudangras).

Als Vergleichsmittel werden folgende aus der EP-A-0 071 707 bekannten Cyclohexan-1,3-dionderivate herangezogen :

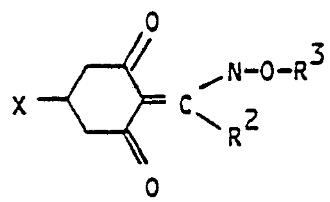

| Nr. | X | $R^2$ | $R^3$ |
|---|---|---|---|
| I | 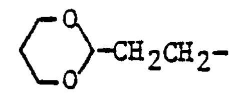 | $n-C_3H_7$ | $C_2H_5$ |
| II | 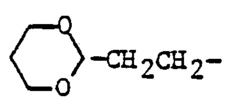 | $n-C_3H_7$ | $-CH_2CH=CH_2$ |
| III | 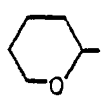 | $n-C_3H_7$ | $C_2H_5$ |
| IV | 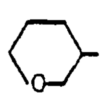 | $n-C_3H_7$ | $C_2H_5$ |
| V | 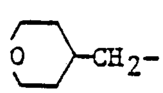 | $n-C_3H_7$ | $-CH_2CH=CH_2$ |
| VI | 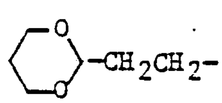 | $n-C_3H_7$ | $-CH_2CH=CHCl$ |

Die Aufwandmengen entsprechen denjenigen der jeweils zu vergleichenden Verbindung der Formel I.

Vorauflaufanwendung :

Bei Vorauflaufanwendung erweisen sich beispielsweise Mittel, die die Verbindungen Nr. 7, 53, 2, 1, 17, 54, 10, 12, 20, 23, 29, 57, 59 oder 60 enthalten, bei einer Aufwandmenge von 3,0 kg Wirkstoff/ha als herbizid wirksam gegen Pflanzen aus der Familie der Gräser. Dabei bleibt Sinapis alba als breitblättrige Testpflanze unbeschadet.

Nachauflaufanwendung :

Bei Nachauflaufanwendung von 0,25 kg Wirkstoff/ha beispielsweise der Verbindungen Nr. 1, 11, 12, 13 und 17 werden bestimmte grasartige unerwünschte Pflanzen oder auch Kulturpflanzen aus der Gräserfamilie, soweit sie an bestimmten Standorten unerwünscht sind, besser bekämpft als mit den Vergleichsmitteln I und III. Sojabohnen als Beispiel für breitblättrige Kulturpflanzen werden dabei nicht geschädigt. Ebenso zeigt die Verbindung Nr. 14 bei einer Aufwandmenge von 0,25 kg Wirkstoff/ha eine dem Vergleichsmittel VI überlegene Wirkung gegen Sorghum halepense bei voller Verträglichkeit für Sojapflanzen.

Die Verbindungen Nr. 2 und 3 zeigen beispielsweise bei einer Aufwandmenge von 0,125 kg Wirkstoff/ha eine sehr gute und den Vergleichsmitteln IV und V überlegene herbizide Wirkung gegen Gramineen, wie Weizen (als Ausfallweizen) und Bromus spp. Die Mittel sind dabei selektiv in Zuckerrüben. Ebenso bekämpfen die Verbindungen Nr. 52 und 53 bei einer Aufwandmenge von 0,125 kg Wirkstoff/ha Grasarten sehr gut. Bei dieser Aufwandmenge zeigen beispielsweise die Verbingungen Nr. 26, 29 und 55 eine gute Wirkung gegen Gräser und verursachen keine Schädigung an Weizen, der durch das Vergleichsmittel I deutliche Schäden erleidet. Mit Verbindung Nr. 7 läßt sich Hühnerhirse als unerwünschte Grasart in Reis bekämpfen, wobei die Kulturpflanze, im Gegensatz zur Verwendung des Vergleichsmittels I, ebenfalls nicht geschädigt wird.

Neben einer Selektivität in breitblättrigen Kulturen zeigt die Verbindung Nr. 6 bei einer niedrigen Aufwandmenge eine beachtliche Wirkung gegen Gräser und verursacht dabei keine Schädigung an Weizen. Das Vergleichsmittel II schädigt dieses Kulturgras stark. Ebenfalls bei geringer Aufwandmenge lassen sich mit Verbindung Nr. 67 unerwünschte Grasarten verschiedenster Vegetationszonen — Ausfallkulturen aus der Familie der Gramineen eingeschlossen — hervorragend bekämpfen. Die herbizide Wirkung der Verbindung Nr. 67 ist der des Vergleichsmittels IV deutlich überlegen.

In Anbetracht der Verträglichkeit und der Vielzahl der Applikationsmethoden können die erfindungsgemäßen Mittel noch in einer weiteren Zahl von Kulturpflanzen zur Beseitigung unerwünschter Wildgräser oder grasartiger Kulturpflanzen, sofern sie an gewissen Standorten unerwünscht sind, eingesetzt werden. In Betracht kommen beispielsweise folgende Kulturen :

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor — Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum | |
| (Gossypium arboreum | Baumwolle |

| | |
|---|---|
| Gossypium herbaceum | |
| Gossypium vitifolium) | |
| Helianthus annuus | Sonnenblume |
| Helianthus tuberosus | Topinanbur |
| Hevea brasiliensis | Parakautschukbaum |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum | Tabak |
| (N. rustica) | |
| Olea europaea | Ölbaum |
| Phaseolus lunatus | Mondbohne |
| Phaseolus mungo | Erdbohne |
| Phaseolus vulgaris | Buschbohnen |
| Petroselinum crispum | Wurzelpetersilie |
| spp. tuberosum | |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais (post directed, only) |

Zur Verbreiterung des Wirkungsspektrums und zur Erzielung synergistischer Effekte können die erfindungsgemäßen Mittel mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate anderer Struktur und andere herbizide Wirkstoffe in Betracht.

Außerdem kann es von Nutzen sein, die neuen Mittel allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Bekämpfung von Ernährungs- und Spurenelementmängeln eingesetzt werden. Es können auch nichtphytotoxische Öle und Ölkonzentrate zugesetzt werden.

**Patentansprüche**

1. Herbizid, enthaltend inerte Zusatzstoffe und ein Cyclohexan-1,3-dion-derivat der Formel I

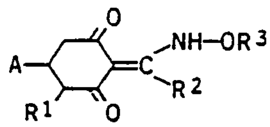

(I)

in der

A Tetrahydropyran-4-yl, 3-Methyltetrahydropyran-4-yl, 1,4-Dioxanyl, 5,5-Dimethyl-1,3-dioxan-2-yl, 2,5-Dimethyl-1,4-dioxan-3-yl, 2,6-Dimethyl-1,4-dioxan-3-yl, 2-Methyl-1,3-dithiolan-2-yl, 2,6-Dimethyltetrahydrothiopyran-3-yl, 2-Methyl-1,3-dithian-2-yl oder gegebenenfalls durch Alkyl mit 1 bis 8 Kohlenstoffatomen, Alkylen mit 4 oder 5 Kohlenstoffatomen oder gegebenenfalls methylsubstituiertes Cycloalkyl oder Bicycloalkyl mit 6 bis 12 Kohlenstoffatomen substituiertes 1,3-Dioxepan-5-yl,

$R^1$ Wasserstoff oder Methoxycarbonyl,

$R^2$ Alkyl mit 1 bis 4 Kohlenstoffatomen und

$R^3$ Alkyl mit bis 4 C-Atomen, Alkenyl mit 3 oder 4 C-Atomen, Halogenalkenyl mit 3 oder 4 C-Atomen und 1 bis 3 Halogensubstituenten oder Propargyl bedeuten,

oder ein Salz einer solchen Verbindung.

2. Herbizid nach Anspruch 1, dadurch gekennzeichnet, daß es 0,1 bis 95 Gew.% des Cyclohexan-1,3-dionderivats enthält.

3. Herbizid nach Anspruch 1, dadurch gekennzeichnet, daß es ein Cyclohexan-1,3-dionderivat der Formel I enthält, wobei A Tetrahydropyran-4-yl, 2,6-Dimethyl-tetrahydrothiopyran-3-yl oder gegebenenfalls durch Alkyl mit 1 bis 8 Kohlenstoffatomen, Alkylen mit 4 oder 5 Kohlenstoffatomen und gegebenenfalls methylsubstituiertes Cycloalkyl oder Bicycloalkyl mit 6 bis 12 Kohlenstoffatomen substituiertes 1,3-Dioxepan-5-yl bedeutet.

4. Verfahren zur Bekämpfung unerwünschten Pflanzenwachstums, dadurch gekennzeichnet, daß man die unerwünschten Pflanzen oder die von unerwünschtem Pflanzenwachstum freizuhaltende Fläche mit einer herbizid wirksamen Menge eines Cyclohexan-1,3-dionderivates der Formel I in Form eines Mittels nach einem der Ansprüche 1 bis 3 behandelt.

**Claims**

1. A herbicide, containing an inert additive and a cyclohexane-1,3-dione derivative of the formula I

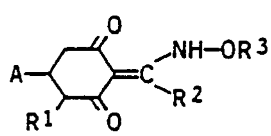

(I)

where A is tetrahydropyran-4-yl, 3-methyltetrahydropyran-4-yl, 1,4-dioxanyl, 5,5-dimethyl-1,3-dioxan-2-yl, 2,5-dimethyl-1,4-dioxan-3-yl, 2,6-dimethyl-1,4-dioxan-3-yl, 2-methyl-1,3-dithiolan-2-yl, 2,6-dimethyltetrahydrothiopyran-3-yl or 2-methyl-1,3-dithian-2-yl, or is 1,3-dioxepan-5-yl which is unsubstituted or substituted by alkyl of 1 to 8 carbon atoms, by alkylene of 4 or 5 carbon atoms or by unsubstituted or methyl-substituted cycloalkyl or bi-cycloalkyl of 6 to 12 carbon atoms, $R^1$ is hydrogen or methoxycarbonyl, $R^2$ is alkyl of 1 to 4 carbon atoms and $R^3$ is alkyl of 1 to 4 carbon atoms, alkenyl of 3 or 4 carbon atoms, haloalkenyl of 3 or 4 carbon atoms and 1 to 3 halogen substituents or propargyl, or a salt thereof.

2. A herbicide as claimed in claim 1, containing from 0.1 to 95 % by weight of the cyclohexane-1,3-dione derivative.

3. A herbicide as claimed in claim 1, containing a cyclohexane-1,3-dione derivative of the formula I where A is tetrahydropyran-4-yl, 2,6-dimethyltetrahydrothiopyran-3-yl or 1,3-dioxepan-5-yl which is unsubstituted or substituted by alkyl of 1 to 8 carbon atoms, alkylene of 4 or 5 carbon atoms, or by unsubstituted or methyl-substituted cycloalkyl or bicycloalkyl of 6 to 12 carbon atoms.

4. A method for controlling undesirable plant growth wherein the undesirable plants or the area to be kept free from undesirable plant growth is treated with a herbicidally effective amount of cyclohexane-1,3-dione derivative of the formula I in the form of a herbicide as claimed in any of claims 1 to 3.

**Revendications**

1. Herbicide, contenant des additifs inertes et un dérivé de cyclohexane-1,3-dione de formule I

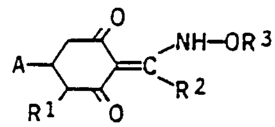 (I)

dans laquelle

A représente tétrahydropyran-4-yle,· 3-méthyltétrahydropyran-4-yle, 1,4-dioxanyle, 5,5-diméthyl-1,3-dioxan-2-yle, 2,5-diméthyl-1,4-dioxan-3-yle, 2,6-diméthyl-1,4-dioxan-3-yle, 2-méthyl-1,3-dithiolan-2-yle, 2,6-diméthyltétrahydrothiopyran-3-yle, 2-méthyl-1,3-dithian-2-yle ou 1,3-dioxepan-5-yle, éventuellement substitué par alkyle à 1 à 8 atomes de carbone, alkylène à 4 ou 5 atomes de carbone ou cycloalkyle ou bicycloalkyle à 6 à 12 atomes de carbone, éventuellement méthylsubstitués,

$R^1$ représente hydrogène ou méthoxycarbonyle,

$R^2$ alkyle à 1 à 4 atomes de carbone et

$R^3$ alkyle à 1 à 4 atomes C, alcényle à 3 ou 4 atomes C, halogénalcényle à 3 ou 4 atomes C et 1 à 3 substituants halogène ou propargyle

ou un sel d'un tel composé.

2. Herbicide selon la revendication 1, caractérisé par le fait qu'il contient 0,1 à 95 % en poids du dérivé de cyclohexane-1,3-dione.

3. Herbicide selon la revendication 1, caractérisé par le fait qu'il contient un dérivé de cyclohexane-1,3-dione de formule I, A représentant tétrahydropyran-4-yle, 2,6-diméthyl-tétrahydrothiopyran-3-yle ou 1,3-dioxepan-5-yle, éventuellement substitué par alkyle à 1 à 8 atomes de carbone, alkylène à 4 ou 5 atomes de carbone ou cycloalkyle ou bicycloalkyle à 6 à 12 atomes de carbone, éventuellement méthylsubstitués.

4. Procédé de lutte contre la croissance des plantes indésirables, caractérisé par le fait que l'on traite les plantes indésirables ou les surfaces à maintenir exemptes d'une croissance indésirable des plantes, avec une quantité efficace du point de vue herbicide d'un dérivé de cyclohexane-1,3-dione de formule I, sous forme d'un moyen selon l'une des revendications 1 à 3.